# EUROPEAN PATENT SPECIFICATION

(11) **EP 4 112 106 B1**
(45) Date of publication and mention of the grant of the patent: **30.07.2025**
(21) Application number: 21776053.7
(22) Date of filing: 10.03.2021
(51) Int. Cl.: A61M 5/32, A61M 5/46, A61M 37/00

(54) **MEDICAL PUNCTURING NEEDLE**
MEDIZINISCHE PUNKTIONSNADEL
AIGUILLE DE PONCTION MÉDICALE

(30) Priority: 24.03.2020 JP 2020052001
(43) Date of publication of application: 04.01.2023
(73) Proprietor: TERUMO Kabushiki Kaisha, Tokyo 151-0072 (JP)
(72) Inventor: IWASE, Yoichiro, Ashigarakami-gun, Kanagawa 259-0151 (JP); MAEKAWA, Minami, Ashigarakami-gun, Kanagawa 259-0151 (JP); YOSHIDA, Yasuyuki, Ashigarakami-gun, Kanagawa 259-0151 (JP)
(74) Representative: Casalonga
(86) International application number: PCT/JP2021/009411
(87) International publication number: WO 2021/193041

(56) References cited:
- WO-A1-2010/007664
- DE-A1- 102006 032 534
- DE-A1- 102006 032 534
- JP-A- 2011 513 036
- US-A1- 2007 256 289
- US-A1- 2009 234 288
- US-A1- 2011 034 885
- US-A1- 2016 199 574

## Description

### Technical Field

The present invention relates to a medical puncture needle for administering a medicine into a living body. In particular, the present invention relates to a medical puncturing needle according to the preamble of claim 1, such as it is e.g. known from US 2016/199574, US 2009 234288 A1 or US 2007 256289 A1.

### Background Art

When a medicine is administered to a thin tissue in a living body such as a gastrointestinal mucous membrane, a posterior eye segment, or a mucous membrane of a nose using a common injection needle, an inclined blade surface may penetrate the tissue to cause a leakage of the liquid medicine to the outside of the tissue, or the injection needle may penetrate through the thin tissue, because the tissue is as thin as 1 mm or less. Therefore, there is a problem that a sufficient amount of liquid medicine cannot be reliably administered to the target tissue.

In order to address such a problem, US 8,562,589 discloses a method of administering a medicine into a gastrointestinal mucous membrane using a puncture needle in which the medicine is applied to the tip of a microneedle.

### Summary of Invention

However, the technology disclosed in US 8,562,589 has a problem that, due to the amount of the medicine applied to the puncture needle being small, a sufficient amount of the medicine cannot be efficiently administered.

In view of this, an object of an embodiment is to provide a medical puncture needle capable of reliably administering a sufficient amount of a liquid medicine to a thin tissue.

In order to solve this problem, the present invention provides a medical puncture needle according to independent claim 1. The dependent claims relate to advantageous embodiments.

According to the medical puncture needle of the above aspect, it is possible to efficiently administer a large amount of liquid medicine to a thin tissue.

### Brief Description of Drawings

Fig. 1A is a perspective view of a medical puncture needle according to a first embodiment, and Fig. 1B is a cross-sectional view of the medical puncture needle illustrated in Fig. 1A.
Fig. 2 is an explanatory diagram illustrating a function of the medical puncture needle according to the first embodiment.
Fig. 3A is an explanatory diagram (part 1) illustrating the action of a medical puncture needle of a comparative example, and Fig. 3B is an explanatory diagram (part 2) illustrating the action of the medical puncture needle of the comparative example.
Fig. 4A is a cross-sectional view (part 1) illustrating a manufacturing process of the medical puncture needle illustrated in Fig. 1A, and Fig. 4B is a cross-sectional view (part 2) illustrating the manufacturing process of the medical puncture needle.
Fig. 5A is a cross-sectional view (part 3) illustrating the manufacturing process of the medical puncture needle illustrated in Fig. 1A, and Fig. 5B is a cross-sectional view (part 4) illustrating the manufacturing process of the medical puncture needle illustrated in Fig. 1A.
Fig. 6A is a perspective view of a medical puncture needle according to a second embodiment, and Fig. 6B is a cross-sectional view of the medical puncture needle illustrated in Fig. 6A.
Fig. 7A is a perspective view of a medical puncture needle according to a third embodiment, and Fig. 7B is a cross-sectional view of the medical puncture needle illustrated in Fig. 7A.
Fig. 8A is a perspective view of a medical puncture needle according to a fourth embodiment, and Fig. 8B is a cross-sectional view of the medical puncture needle illustrated in Fig. 8A.
Fig. 9A is a perspective view of a medical puncture needle according to a fifth embodiment, and Fig. 9B is a cross-sectional view of the medical puncture needle illustrated in Fig. 9A.

### Description of Embodiments

Preferred embodiments of the present invention will now be described in detail with reference to the drawings.

### (First Embodiment)

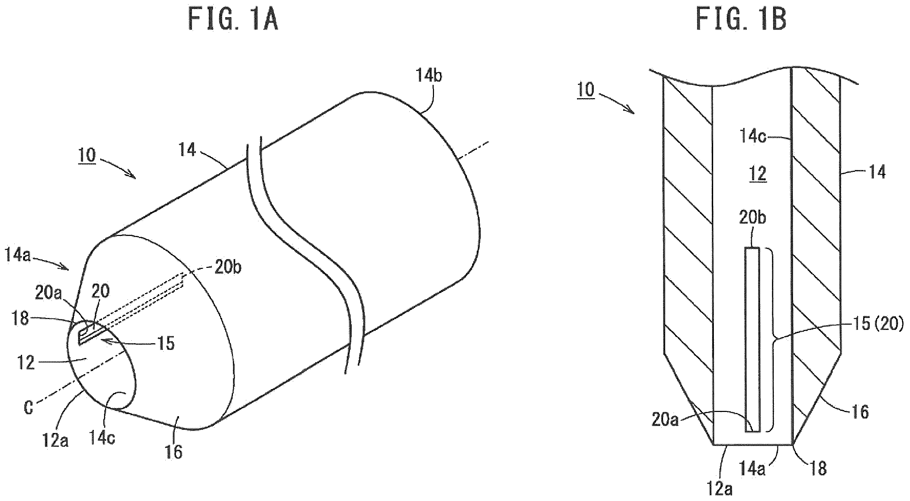

As illustrated in Fig. 1A, a medical puncture needle 10 according to the present embodiment includes a cylindrical main body 14 having a through hole 12 formed therein, a blade surface 16 provided at a needle tip 14a of the main body 14, and a circular blade edge 18 formed at a tip of the blade surface 16.

The main body 14 is formed in a long cylindrical shape and is made of, for example, a metal material such as stainless steel. The diameter (outer diameter) of the main body 14 can be set to, for example, about 36 G (about 0.1 mm).

The through hole 12 extends in the axial direction along a central axis C of the main body 14. The through hole 12 is formed as a cavity having a circular cross section, and is formed to penetrate from a proximal end 14b of the main body 14 to the needle tip 14a. When the diameter of the main body 14 is set to, for example, 36 G (about 0.1 mm), the inner diameter of the through hole 12 can be set to, for example, about 0.05 mm. An opening part 12a at the tip of the through hole 12 is provided inside the circular blade edge 18. That is, the opening part 12a of the through hole 12 is formed along a plane perpendicular to the central axis C of the medical puncture needle 10.

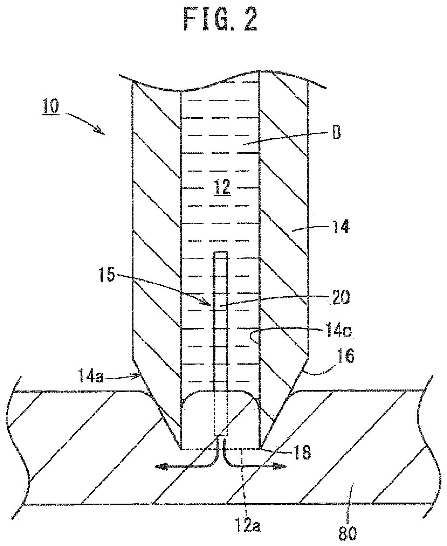

A liquid medicine permeation portion 15 is formed on an inner peripheral surface 14c of the through hole 12 near the needle tip 14a. The liquid medicine permeation portion 15 of the present embodiment has a liquid medicine passage groove 20 extending in the direction of the central axis C. The liquid medicine passage groove 20 serves as a flow path for guiding the liquid medicine to the needle tip 14a when the through hole 12 is closed by coring on a tissue 80 (see Fig. 2) due to insertion of the medical puncture needle 10 into the tissue 80. The liquid medicine passage groove 20 is formed as a groove-shaped recess that is recessed outward with respect to the inner peripheral surface 14c, and linearly extends in the direction of the central axis C. A tip part 20a of the liquid medicine passage groove 20 is positioned in the vicinity of the tip of the through hole 12.

In addition, the liquid medicine passage groove 20 has a width and a depth capable of inhibiting entry of the tissue 80 during coring. When the diameter (outer diameter) of the main body 14 is set to, for example, 36 G (about 0.10 mm) to 38 G (about 0.05 mm), the width (dimension in the circumferential direction) of the liquid medicine passage groove 20 can be set to, for example, about 0.025 to 0.070 mm, and the depth (dimension in the radial direction) of the liquid medicine passage groove 20 can be set to about 0.005 to 0.015 mm.

In addition, when the diameter (outer diameter) of the main body 14 is set to, for example, 22 G (about 0.70 mm) to 34 G (about 0.18 mm), the width (dimension in the circumferential direction) of the liquid medicine passage groove 20 can be set to, for example, about 0.060 to 0.535 mm, and the depth (dimension in the radial direction) of the liquid medicine passage groove 20 can be set to about 0.005 to 0.145 mm. when the liquid medicine is administered to a site where coring is likely to occur or when the liquid medicine has high viscosity, it is preferable that the diameter (outer diameter) of the main body 14 is large, the width of the liquid medicine passage groove 20 is large, and the depth of the liquid medicine passage groove 20 is small.

Further, the position of a proximal end 20b of the liquid medicine passage groove 20 illustrated in Fig. 1B extends to a position where the tissue 80 (see Fig. 2) of the living body cannot enter. The proximal end 20b of the liquid medicine passage groove 20 is preferably located, for example, at a position 2.5 mm or more away from the opening part 12a toward the proximal end side.

A plurality of the liquid medicine passage grooves 20 may be provided in the inner peripheral surface 14c of the through hole 12 in the circumferential direction. In a case where two liquid medicine passage grooves 20 are provided, the liquid medicine passage grooves 20 are arranged at an angle of 180° in the circumferential direction. In addition, three, four, or more liquid medicine passage grooves 20 may be provided. In a case where the plurality of liquid medicine passage grooves 20 is provided, it is preferable to arrange the liquid medicine passage grooves evenly in the circumferential direction, and the plurality of liquid medicine passage grooves can be equally spaced in the circumferential direction.

As illustrated in Fig. 1A, the blade surface 16 is formed as an inclined curved surface obtained by obliquely cutting the needle tip 14a of the cylindrical main body 14 from the outer peripheral side toward the center. As illustrated in Fig. 1B, the cross section of the main body 14 at the portion where the blade surface 16 is formed is tapered so that the thickness is decreased toward the tip. The angle (inclination angle) between the blade surface 16 and the central axis C (see Fig. 1A) of the main body 14 can be set to, for example, about 8° to 75°.

The blade edge 18 is sharply formed at a portion where the blade surface 16 and the inner peripheral surface 14c of the through hole 12 are in contact with each other, and can puncture the tissue 80 of the living body. The blade edge 18 is formed in an annular shape along a plane perpendicular to the central axis C of the main body 14. With this configuration, a load is distributed to the entire region of the blade edge 18 during puncturing. Therefore, the blade edge 18 is difficult to penetrate the tissue 80, so that the blade edge 18 easily punctures the tissue 80 shallowly.

The medical puncture needle 10 described above is used in such a manner that the main body 14 is inserted into the thin layered tissue 80 in a direction perpendicular to the tissue 80 (thickness direction of the tissue 80) as illustrated in Fig. 2. Examples of the thin layered tissue 80 include intestinal mucosa, posterior eye segment, and olfactory mucosa, and the thickness of the tissue 80 is 1 mm or less. When the medical puncture needle 10 is inserted in the manner described above, the entire region of the blade edge 18 is aligned in the layer direction of the thin tissue 80, so that the entire region of the opening part 12a of the through hole 12 stays in the tissue 80. With the tissue 80 being punctured by the blade edge 18, a part of the tissue 80 enters the inside of the through hole 12 by the coring. Therefore, most of the through hole 12 is closed by the tissue 80. It is to be noted, however, that the tissue 80 cannot enter the liquid medicine passage groove 20, because the liquid medicine passage groove 20 has a fine groove shape. Therefore, the liquid medicine passage groove 20 forms a flow path through which the liquid medicine can pass.

The liquid medicine is guided to the vicinity of the needle tip 14a of the medical puncture needle 10 through the liquid medicine passage groove 20, and is injected into the tissue 80 from the vicinity of the blade edge 18. As a result, the medical puncture needle 10 can efficiently administer a large amount of liquid medicine to the extremely thin tissue 80.

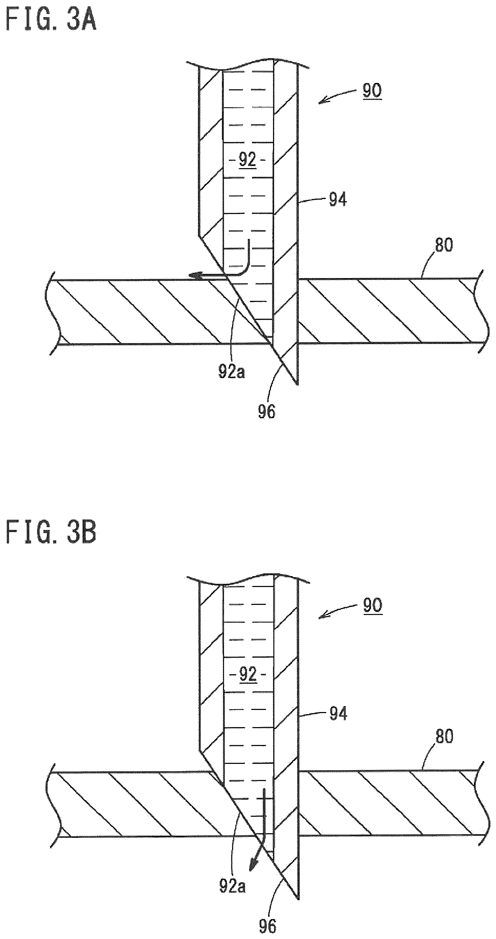

As illustrated in Fig. 3A, in a medical puncture needle 90 of a comparative example, a blade surface 96 has a plane inclined with respect to the axial direction, and an opening part 92a of a through hole 92 of a needle tube 94 is exposed to the plane inclined in the axial direction. The opening part 92a of the through hole 92 is elongated along the axial direction of the medical puncture needle 90. Therefore, when the medical puncture needle 90 is inserted, a part of the opening part 92a of the through hole 92 on the proximal end side protrudes from the thin tissue 80, and this causes a leakage of the liquid medicine.

In addition, as illustrated in Fig. 3B, when the medical puncture needle 90 of the comparative example is deeply inserted, the opening part 92a of the through hole 92 penetrates the tissue 80 to cause a leakage of the liquid medicine. Therefore, the medical puncture needle 90 of the comparative example cannot efficiently administer the liquid medicine to the thin tissue 80.

On the other hand, in the medical puncture needle 10 according to the present embodiment, the opening part 12a of the through hole 12 is formed inside the blade edge 18 perpendicular to the axial direction of the medical puncture needle 10, and thus is short in the axial direction. Therefore, even when the very thin tissue 80 is punctured, the opening part 12a does not penetrate the tissue 80, and the liquid medicine can be injected into the tissue 80 without leakage.

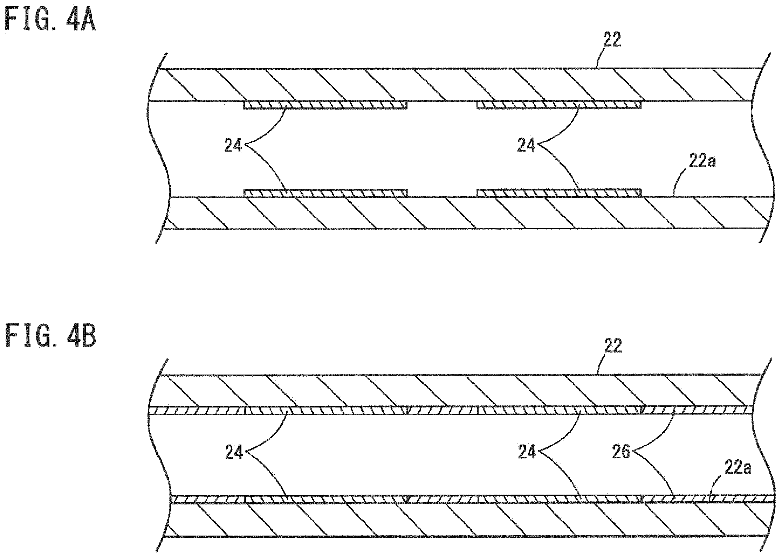

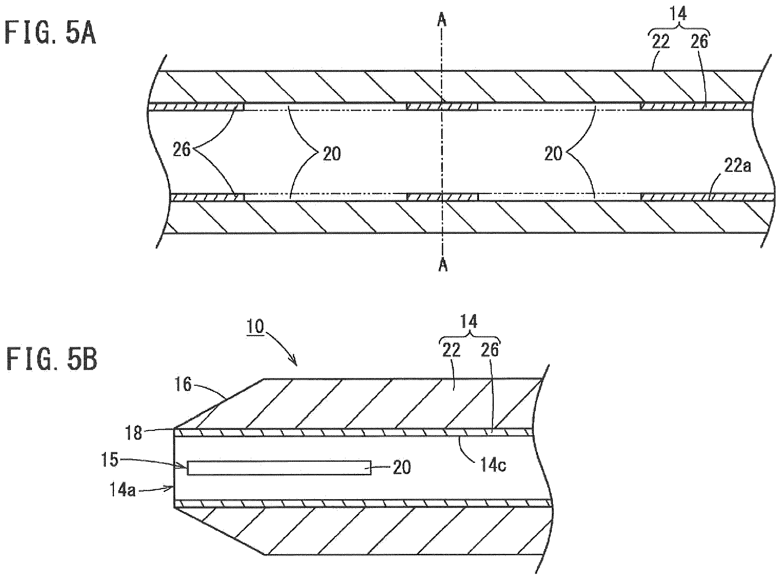

The medical puncture needle 10 described above can be manufactured in such a manner that, as illustrated in Figs. 4A to 5B, a metal tube 22 constituting the main body 14 is prepared, and the outer peripheral portion of the needle tip 14a of the main body 14 is electrolytically polished in a tapered shape to form the blade surface 16 and the blade edge 18. In addition, the liquid medicine passage groove 20 can be formed by performing various types of processing, for example, machining (cutting or pressing), on the inner peripheral wall of the main body 14.

The liquid medicine passage groove 20 of the medical puncture needle 10 is formed using plating. The case where the liquid medicine passage groove 20 of the medical puncture needle 10 is formed by plating will be described below.

As illustrated in Fig. 4A, first, the metal tube 22 is formed as a material of the medical puncture needle 10. Next, a mask 24 made of, for example, a resin material is formed on a part of an inner surface 22a of the metal tube 22 by coating. The mask 24 is formed in the same planar shape as the liquid medicine passage groove 20. Prior to the formation of the mask 24, a seed layer serving as a base of the plating film may be formed.

Next, as illustrated in Fig. 4B, a metal layer 26 is formed inside the metal tube 22 by a method such as electrolytic plating or electroless plating. The metal layer 26 grows with the inner surface 22a of a portion not covered with the mask 24 as a base. The inner surface 22a of the portion not covered with the mask 24 is covered with the metal layer 26. The metal layer 26 is made of, for example, metal such as Ni/Cr alloy, Cu/Cr alloy, Ni, Ni base alloy, Fe, Fe base alloy, Co, Co base alloy, Ag, Ag base alloy, Cu, Cu base alloy, Au, and Au base alloy.

Thereafter, the mask 24 is removed as illustrated in Fig. 5A. The mask 24 can be removed using a solvent, or the like. Due to the removal of the mask 24, the liquid medicine passage groove 20 having a recessed groove is formed in the metal tube 22. Next, the A-A portion in Fig. 5A is cut. Thus, two main bodies 14 having the cut portions of the metal tube 22 as tips are obtained.

Next, as illustrated in Fig. 5B, the blade surface 16 and the blade edge 18 are formed by performing electrolytic polishing obliquely along the outer periphery of the tip of the main body 14. Through the above steps, the medical puncture needle 10 according to the present embodiment is completed.

The medical puncture needle 10 according to the present embodiment has the following effects.

The medical puncture needle 10 according to the present embodiment includes: the cylindrical main body 14 having the through hole 12 formed therein; the annular blade surface 16 cut into a tapered shape so that the thickness of the main body 14 reduces toward the needle tip 14a of the main body 14; and the circular blade edge 18 formed at the tip of the blade surface 16 so as to extend along a plane orthogonal to the central axis C of the main body 14.

According to the above configuration, when the medical puncture needle 10 is inserted into the thin tissue 80 in the direction perpendicular to the tissue 80, the through hole 12 does not penetrate the tissue 80, so that a large amount of liquid medicine can be efficiently injected into the tissue 80 without causing leakage of the liquid medicine.

In the medical puncture needle 10 described above, the liquid medicine permeation portion 15 having an uneven structure may be further formed in the inner peripheral surface 14c of the through hole 12 in the vicinity of the blade edge 18.

When the medical puncture needle 10 is perpendicularly inserted into the tissue 80, the through hole 12 may be closed by the tissue 80 due to coring. Even in such a case, it is possible to allow the liquid medicine to flow toward the blade edge 18 through the liquid medicine permeation portion 15, whereby a sufficient amount of liquid medicine can be injected into the tissue 80.

In the medical puncture needle 10 described above, the liquid medicine permeation portion 15 may include the liquid medicine passage groove 20 having a groove-shaped recess extending in the axial direction. Accordingly, even when the through hole 12 is closed by coring, it is possible to allow the liquid medicine to flow toward the blade edge 18 through the liquid medicine passage groove 20.

In the medical puncture needle 10 described above, a plurality of the liquid medicine passage grooves 20 may be provided in the inner peripheral surface 14c of the through hole 12. With this configuration, even when coring occurs, a sufficient amount of the liquid medicine can be injected into the tissue 80.

### (Second Embodiment)

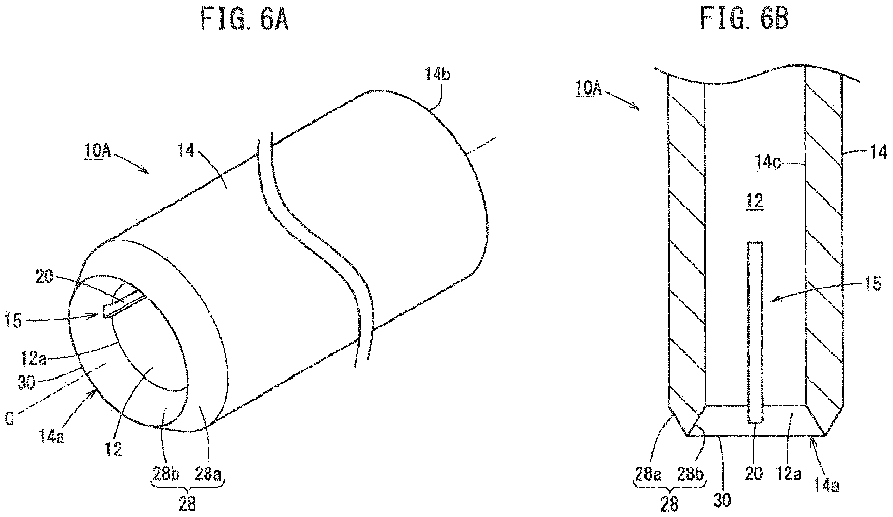

As illustrated in Fig. 6A, a medical puncture needle 10A according to the present embodiment is different from the medical puncture needle 10 in Fig. 1A in the shape of a blade surface 28 formed at a needle tip 14a of a cylindrical main body 14. Note that the components of the medical puncture needle 10A similar to the components of the medical puncture needle 10 in Fig. 1A are denoted by the same reference numerals, and the detailed description thereof will be omitted.

As illustrated in Fig. 6B, in the medical puncture needle 10A, the blade surface 28 includes an outer blade surface 28a obtained by obliquely cutting the main body 14 from the outer peripheral side, and an inner blade surface 28b obtained by obliquely cutting the main body 14 from the inner peripheral side. The outer blade surface 28a and the inner blade surface 28b have annular curved surfaces, and a sharp blade edge 30 is formed at a tip portion where the outer blade surface 28a and the inner blade surface 28b meet. The outer blade surface 28a and the inner blade surface 28b can be formed by electrolytic polishing.

As illustrated in Fig. 6A, the blade edge 30 is formed in an annular shape along a plane perpendicular to the central axis C of the main body 14. The blade edge 30 in the present embodiment is wider than the through hole 12 of the main body 14, and has a larger diameter than the blade edge 18 in Fig. 1A. According to the medical puncture needle 10A of the present embodiment, a load applied during puncture of the tissue 80 is distributed to the blade edge 30 in a wider area, whereby the liquid medicine easily flows out from the gap between the blade edge 30 and the tissue 80.

As described above, in the medical puncture needle 10A according to the present embodiment, the blade surface 28 is formed on both the inner peripheral side and the outer peripheral side of the main body 14. As a result, even in a case where the coring occurs, the load applied during puncture of the tissue 80 is distributed to the blade edge 30 in a wider area, so that the liquid medicine easily flows out from the gap between the blade edge 30 and the tissue 80.

### (Third Embodiment)

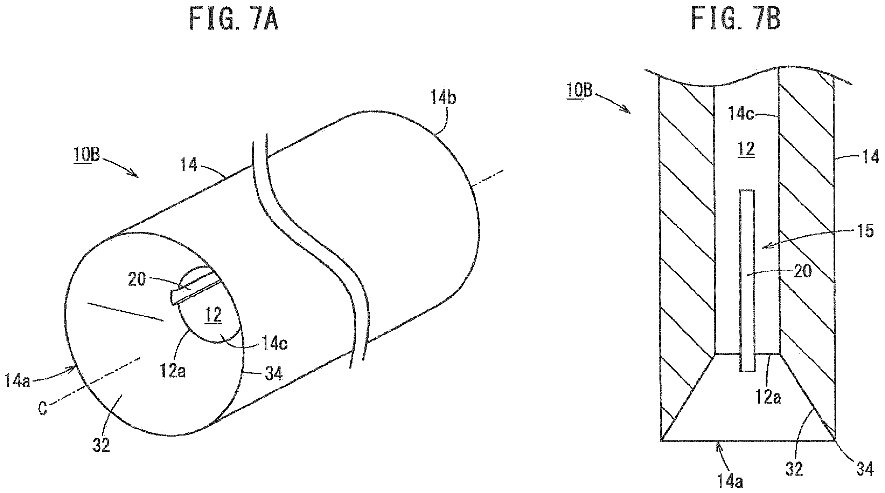

As illustrated in Fig. 7A, a medical puncture needle 10B according to the present embodiment is different from the medical puncture needle 10 in Fig. 1A in the shape of a blade surface 32 formed at a needle tip 14a of a cylindrical main body 14. Note that the components of the medical puncture needle 10B similar to the components of the medical puncture needle 10 in Fig. 1A are denoted by the same reference numerals, and the detailed description thereof will be omitted.

As illustrated in Fig. 7B, in the medical puncture needle 10B, the blade surface 32 is formed by cutting the main body 14 in a tapered shape from the inner peripheral side. The blade surface 32 has an annular curved surface obliquely cut along the circumferential direction of the main body 14. A sharp blade edge 34 is formed on a side where the outer peripheral surface of the main body 14 and the blade surface 32 meet. The blade edge 34 in the present embodiment is wider than the through hole 12 of the main body 14, and has a diameter equal to the diameter of the main body 14.

As described above, in the medical puncture needle 10B according to the present embodiment, the blade surface 32 is formed only on the inner peripheral side of the main body 14. Thus, the blade edge 34 widens to have a diameter substantially equal to the diameter of the main body 14. As a result, even in a case where the coring occurs when the medical puncture needle 10B is inserted into the tissue 80, the load applied during insertion into the tissue 80 is distributed to the blade edge 34 in a wider area, so that the liquid medicine easily flows out from the gap between the blade edge 34 and the tissue 80. As a result, the medical puncture needle 10B can inject a large amount of liquid medicine into the tissue 80.

### (Fourth Embodiment)

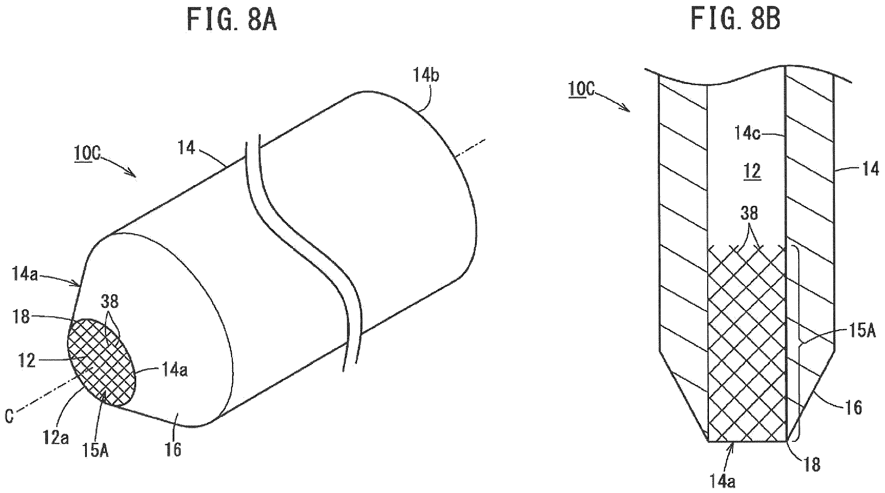

As illustrated in Figs. 8A and 8B, a medical puncture needle 10C according to the present embodiment is different from the medical puncture needle 10 in Fig. 1A in a liquid medicine permeation portion 15A formed in an inner peripheral surface 14c of a through hole 12 of a main body 14. Note that the components of the medical puncture needle 10C similar to the components of the medical puncture needle 10 in Fig. 1A are denoted by the same reference numerals, and the detailed description thereof will be omitted.

In the medical puncture needle 10C according to the present embodiment, the liquid medicine permeation portion 15A having an uneven structure is formed in the inner peripheral surface 14c of the through hole 12. The liquid medicine permeation portion 15A is formed by a plurality of liquid medicine passage grooves 38 arranged in a matrix. The liquid medicine passage grooves 38 can be formed by changing the pattern of the mask 24 in the step of forming the liquid medicine passage groove 20 by plating described with reference to Figs. 4A to 5B.

According to the medical puncture needle 10C of the present embodiment, even if coring occurs during puncture of the tissue 80, it is possible to allow the liquid medicine to flow toward the needle tip 14a of the main body 14 through the liquid medicine permeation portion 15A. As a result, the medical puncture needle 10C can inject a large amount of liquid medicine into the tissue 80.

Note that the structure of the liquid medicine permeation portion 15A of the present embodiment is not limited to the liquid medicine passage grooves 38 in a matrix, and can be replaced with various uneven structures such as protrusions that can generate a gap between the tissue 80 and the inner peripheral surface 14c of the through hole 12 when coring occurs.

### (Fifth Embodiment)

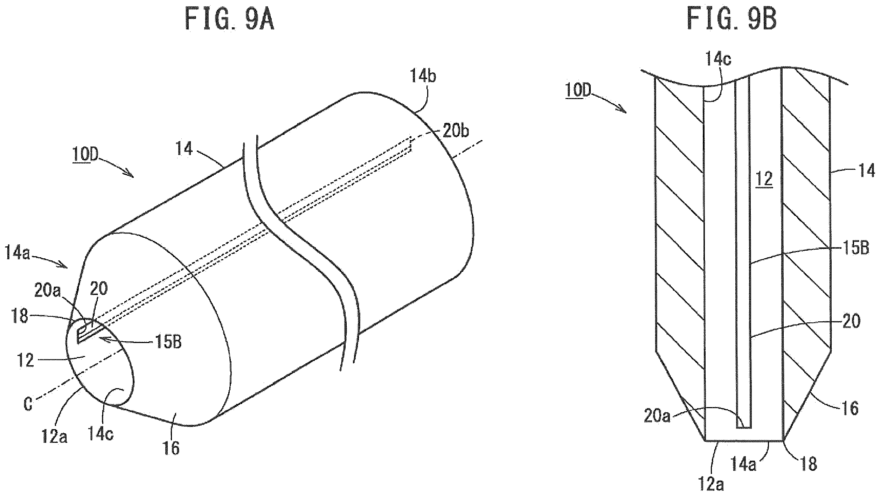

As illustrated in Figs. 9A and 9B, a medical puncture needle 10D according to the present embodiment is different from the medical puncture needle 10 in Fig. 1A in a liquid medicine permeation portion 15B formed in an inner peripheral surface 14c of a through hole 12 of a main body 14. Note that the components of the medical puncture needle 10D similar to the components of the medical puncture needle 10 in Fig. 1A are denoted by the same reference numerals, and the detailed description thereof will be omitted.

The medical puncture needle 10D according to the present embodiment has the liquid medicine permeation portion 15B which is formed in the inner peripheral surface 14c of the through hole 12 and which has a liquid medicine passage groove 20 extending to a proximal end 14b of the medical puncture needle 10D. The medical puncture needle 10D according to the present embodiment is manufactured as follows. First, a core material (not illustrated) having the same outer peripheral surface shape as the shape of the inner peripheral surface 14c of the through hole 12 and the liquid medicine permeation portion 15B is prepared.

The core material is formed with a linear protrusion extending in the axial direction in a portion corresponding to the liquid medicine passage groove 20. Next, the core material is immersed in a plating solution (containing, for example, ions of Ni/Cr, Cu/Cr, Ni, Fe, Co, Ag, Cu, Au, etc.), and an electroformed body (for example, an electroformed body of Ni/Cr, Cu/Cr, Ni, Fe, Co, Ag, Cu, Au, etc.) is formed on the outer peripheral surface of the core material by plating. An electrolytic plating method is preferably used for forming the electroformed body by plating, but an electroless plating method may be partly used in combination during the process. Thereafter, the core material is removed from the electroformed body from the proximal end side to obtain the main body 14 constituted by a tubular electroformed body. The liquid medicine passage groove 20 is formed in the inner peripheral surface 14c of the main body 14. Then, the blade surface 16 and the blade edge 18 are formed by performing electrolytic polishing obliquely along the outer periphery of the tip of the main body 14. Through the above steps, the medical puncture needle 10D according to the present embodiment is completed.

The medical puncture needles in the second embodiment and the third embodiment described above can also be manufactured in the same manner.

While the present invention has been described above with reference to preferred embodiments, it is obvious that the present invention is not limited to the above embodiments, and that various modifications are possible without departing from the scope of the present invention.

## Claims

1. A medical puncture needle (10, 10A-D, 90) comprising:
a main body (14) having a cylindrical shape and having inside a through hole (12, 92);
a blade surface (16, 28, 28a-b, 32, 96) that has an annular shape and that is cut into a tapered shape so that a thickness of the main body (14) reduces toward a tip part (20a) of the main body (14); and
a blade edge (18, 30, 34) that has an annular shape, the blade edge (18, 30, 34) being formed at a tip of the blade surface (16, 28, 28a-b, 32, 96) so as to extend along a plane orthogonal to a central axis (C) of the main body (14),
**wherein**
the medical puncture needle (10, 10A-D, 90) is configured for administering a liquid medicine into a tissue (80) of a living body,
the blade edge (18, 30, 34) is sharp, and
the diameter of the main body (14) is 22 G to 34 G,
**characterized in that**
the medical puncture needle (10, 10A-D, 90) further comprises:
a liquid medicine permeation portion (15, 15A-B) formed on an inner peripheral surface (14c) of the through hole (12, 92) near the blade edge (18, 30, 34), the liquid medicine permeation portion (15, 15A-B) guiding the liquid medicine to the blade edge (18, 30, 34) when the through hole (12, 92) is closed by the tissue (80),
wherein
the liquid medicine permeation portion (15, 15A-B) comprises a liquid medicine passage groove (20, 38) having a groove-shaped recess extending in an axial direction, or a protrusion generating a gap between the tissue (80) and the inner peripheral surface (14c),
the liquid medicine passage groove (20, 38) has a width and a depth inhibiting entry of the tissue (80),
a proximal end of the liquid medicine passage groove (20, 38) extends to a position where the tissue (80) does not enter, and
the liquid medicine passage groove (20, 38) is formed using plating.

2. The medical puncture needle (10, 10A-D, 90) according to claim 1, wherein a plurality of the liquid medicine passage grooves (20, 38) is provided in the inner peripheral surface (14c) of the through hole (12, 92).

3. The medical puncture needle (10, 10A-D, 90) according to any one of claims 1 to 2, wherein the blade surface (16, 28, 28a-b, 32, 96) is provided on an outer peripheral side of the main body (14).

4. The medical puncture needle (10, 10A-D, 90) according to any one of claims 1 to 2, wherein the blade surface (16, 28, 28a-b, 32, 96) is provided on both an inner peripheral side and an outer peripheral side of the main body (14).

5. The medical puncture needle (10, 10A-D, 90) according to any one of claims 1 to 2, wherein the blade surface (16, 28, 28a-b, 32, 96) is provided only on an inner peripheral side of the main body (14).

## Patentansprüche

1. Medizinische Punktionsnadel (10, 10A-D, 90) umfassend:
einen Hauptkörper (14), der eine zylindrische Form besitzt und im Inneren ein Durchgangsloch (12, 92) aufweist;
eine Schneidfläche (16, 28, 28a-b, 32, 96), die eine ringförmige Form aufweist und die in eine sich verjüngende Form geschnitten ist, sodass eine Dicke des Hauptkörpers (14) sich in Richtung eines Spitzenteils (20a) des Hauptkörpers (14) verringert; und
eine Schneidkante (18, 30, 34), die eine ringförmige Form aufweist, wobei die Schneidkante (18, 30, 34) an einer Spitze der Schneidfläche (16, 28, 28a-b, 32, 96) so ausgebildet ist, dass sie sich entlang einer Ebene orthogonal zu einer Mittelachse (C) des Hauptkörpers (14) erstreckt,
**wobei**
die medizinische Punktionsnadel (10, 10A-D, 90) für die Verabreichung eines flüssigen Medikaments in ein Gewebe (80) eines lebenden Körpers konfiguriert ist,
die Schneidkante (18, 30, 34) scharf ist, und
der Durchmesser des Hauptkörpers (14) 22 G bis 34 G beträgt,
**dadurch gekennzeichnet, dass**
die medizinische Punktionsnadel (10, 10A-D, 90) ferner umfasst:
einen Durchdringungsabschnitt (15, 15A-B) für ein flüssiges Medikament, der an einer inneren Umfangsfläche (14c) des Durchgangslochs (12, 92) in der Nähe der Schneidkante (18, 30, 34) ausgebildet ist, wobei der Durchdringungsabschnitt (15, 15A-B) für ein flüssiges Medikament das flüssige Medikament zu der Schneidkante (18, 30, 34) leitet, wenn das Durchgangsloch (12, 92) durch das Gewebe (80) verschlossen ist,
wobei
der Durchdringungsabschnitt (15, 15A-B) für ein flüssiges Medikament eine Durchgangsnut (20, 38) für ein flüssiges Medikament umfasst, die eine rillenförmige Vertiefung, welche sich in einer axialen Richtung erstreckt, oder einen Vorsprung aufweist, der einen Spalt zwischen dem Gewebe (80) und der inneren Umfangsfläche (14c) erzeugt,
die Durchgangsnut (20, 38) für ein flüssiges Medikament eine Breite und eine Tiefe aufweist, die das Eindringen des Gewebes (80) verhindert,
ein proximales Ende der Durchgangsnut (20, 38) für ein flüssiges Medikament sich zu einer Position erstreckt, wo das Gewebe (80) nicht eindringt, und
die Durchgangsnut (20, 38) für ein flüssiges Medikament unter Verwendung von Plattieren gebildet wird.

2. Medizinische Punktionsnadel (10, 10A-D, 90) nach Anspruch 1, wobei eine Mehrzahl der Durchgangsrillen (20, 38) für ein flüssiges Medikament in der inneren Umfangsfläche (14c) des Durchgangslochs (12, 92) vorgesehen ist.

3. Medizinische Punktionsnadel (10, 10A-D, 90) nach einem der Ansprüche 1 bis 2, wobei die Schneidfläche (16, 28, 28a-b, 32, 96) an einer äußeren Umfangsseite des Hauptkörpers (14) vorgesehen ist.

4. Medizinische Punktionsnadel (10, 10A-D, 90) nach einem der Ansprüche 1 bis 2, wobei die Schneidfläche (16, 28, 28a-b, 32, 96) sowohl auf einer inneren Umfangsseite als auch auf einer äußeren Umfangsseite des Hauptkörpers (14) vorgesehen ist.

5. Medizinische Punktionsnadel (10, 10A-D, 90) nach einem der Ansprüche 1 bis 2, wobei die Schneidfläche (16, 28, 28a-b, 32, 96) nur an einer inneren Umfangsseite des Hauptkörpers (14) vorgesehen ist.

## Revendications

1. Aiguille de ponction médicale (10, 10A-D, 90) comprenant :
un corps principal (14) ayant une forme cylindrique et ayant à l'intérieur un trou traversant (12, 92) ;
une surface de lame (16, 28, 28a-b, 32, 96) qui a une forme annulaire et qui est découpée en une forme conique de sorte qu'une épaisseur du corps principal (14) soit réduite en direction d'une partie de pointe (20a) du corps principal (14) ; et
un bord de lame (18, 30, 34) qui a une forme annulaire, le bord de lame (18, 30, 34) étant formé au niveau d'une pointe de la surface de lame (16, 28, 28a-b, 32, 96) de manière à s'étendre le long d'un plan orthogonal à un axe central (C) du corps principal (14),
**dans laquelle**
l'aiguille de ponction médicale (10, 10A-D, 90) est conçue pour administrer un médicament liquide dans un tissu (80) d'un corps vivant,
le bord de lame (18, 30, 34) est tranchant, et
le diamètre du corps principal (14) est de 22 G à 34 G,
**caractérisée en ce que**
l'aiguille de ponction médicale (10, 10A-D, 90) comprend en outre :
une partie de perméation de médicament liquide (15, 15A-B) formée sur une surface périphérique interne (14c) du trou traversant (12, 92) près du bord de lame (18, 30, 34), la partie de perméation de médicament liquide (15, 15A-B) guidant le médicament liquide vers le bord de lame (18, 30, 34) lorsque le trou traversant (12, 92) est fermé par le tissu (80),
dans laquelle
la partie de perméation de médicament liquide (15, 15A-B) comprend une rainure de passage de médicament liquide (20, 38) présentant un évidement en forme de rainure s'étendant dans une direction axiale, ou une saillie générant un espace entre le tissu (80) et la surface périphérique interne (14c),
la rainure de passage de médicament liquide (20, 38) a une largeur et une profondeur empêchant l'entrée du tissu (80), une extrémité proximale de la rainure de passage de médicament liquide (20, 38) s'étend jusqu'à une position où le tissu (80) n'entre pas, et
la rainure de passage de médicament liquide (20, 38) est formée à l'aide d'un placage.

2. Aiguille de ponction médicale (10, 10A-D, 90) selon la revendication 1, dans laquelle une pluralité de rainures de passage de médicament liquide (20, 38) est disposée dans la surface périphérique interne (14c) du trou traversant (12, 92).

3. Aiguille de ponction médicale (10, 10A-D, 90) selon l'une quelconque des revendications 1 et 2, dans laquelle la surface de lame (16, 28, 28a-b, 32, 96) est disposée sur un côté périphérique externe du corps principal (14).

4. Aiguille de ponction médicale (10, 10A-D, 90) selon l'une quelconque des revendications 1 et 2, dans laquelle la surface de lame (16, 28, 28a-b, 32, 96) est disposée à la fois sur un côté périphérique interne et un côté périphérique externe du corps principal (14).

5. Aiguille de ponction médicale (10, 10A-D, 90) selon l'une quelconque des revendications 1 et 2, dans laquelle la surface de lame (16, 28, 28a-b, 32, 96) est disposée uniquement sur un côté périphérique interne du corps principal (14).
